# EUROPEAN PATENT APPLICATION

(11) **EP 4 653 541 A2**
(43) Date of publication of application: **26.11.2025**
(21) Application number: 25206060.3
(22) Date of filing: 26.10.2021
(51) Int. Cl.: C12P 21/02

(54) **MODIFIED SIGNAL PEPTIDE**

(30) Priority: 28.10.2020 JP 2020180382
(62) Divisional of application: 21886174.8
(71) Applicant: KAO CORPORATION, Tokyo 103-8210 (JP)
(72) Inventor: NISHIGUCHI, Hiroki, Wakayama-shi 640-8580 (JP); KAWAHARA, Akihito, Wakayama-shi 640-8580 (JP)
(74) Representative: Vossius & Partner Patentanwälte Rechtsanwälte mbB

(57) **Abstract**

A nucleic acid construct includes a polynucleotide encoding a protein of interest and a polynucleotide linked upstream of the polynucleotide and encoding a modified signal peptide. The modified signal peptide includes amino acid residues of X₁X₂X₃X₄X₅X₆ at positions 1 to 6 as counted from the C-terminus, where X₁ is G, P, or A; X₂ is G, S, or A; and X₃ is P, S, or A, provided that X₁X₂X₃ is not GGP; X₄ is A; X₅ is S, H, or F; and X₆ is A and is located at the C-terminus of the modified signal peptide.

## Description

### Field of the Invention

The present invention relates to a modified signal peptide.

### Background of the Invention

*Bacillus* bacteria such as *Bacillus subtilis* have a function of secreting and producing proteins such as a protease and are therefore suitable as hosts for microbiological production of materials of interest. A secretory protein in *Bacillus* bacteria is expressed as a preproprotein having a signal peptide and is secreted extracellularly by the action of the signal peptide. The signal peptide is cleaved by a signal peptidase, and the preproprotein is then secreted extracellularly and eventually becomes a mature protein. Technology of linking a *Bacillus* bacterium-derived signal peptide to a protein of interest and secreting and producing extracellularly the protein has been used. For example, the signal peptide of a cellulase derived from *Bacillus* sp. KSM-S237 strain (Patent Literature 1) is a signal peptide suitable for efficient secretory production of a protein of interest.

A signal peptide generally consists of a positively charged N-terminal region, a hydrophobic transmembrane region (H region) forming an α helix, and a C-terminal region having a sheet-like structure. The peptide in the C-terminal region is mainly cleaved by a signal peptidase. It has been reported that modification in the signal peptide may affect the protein productivity (for example, Non Patent Literature 1).

Small molecule antibodies have advantages such as production efficiency, ease of drug design, and tissue penetrability and are therefore attracting attention as materials for antibody drugs and reagents. Typical examples of the small molecule antibody include a Fab, a single chain antibody (scFv), a diabody, and a variable domain of heavy chain of heavy chain antibody (VHH). Conventionally, *Escherichia coli* has been mainly used in microbiological production of antibodies. On the other hand, Non Patent Literature 2 describes that an scFv, a Fab, and a VHH could be produced by a *Brevibacillus* expression system. Non Patent Literature 3 reports that a VHH was produced using *Aspergillus awamori.* Non Patent Literature 4 describes that a *Bacillus subtilis* strain coexpressing intracellular and extracellular molecular chaperones and having inactivated cell wall-binding protease WprA secreted and produced an scFv derived from a fibrin-specific monoclonal antibody.

### (Patent Literature 1) JP-A-2000-210081

(Non Patent Literature 1) Protein Sci., 2012, 21(1): 13-25
(Non Patent Literature 2) Biotechnology (Seibutsu Kogaku), 2017, 95(11): 649-651
(Non Patent Literature 3) Appl. Microbiol. Biotechnol., 2005, 66(4): 384-392
(Non Patent Literature 3) Appl. Environ. Microbiol., 2002, pp. 3261-3269

### Summary of the Invention

In one aspect, the present invention provides a nucleic acid construct for expression and extracellular secretion of a protein of interest, comprising a polynucleotide encoding the protein of interest and a polynucleotide linked upstream of the polynucleotide and encoding a modified signal peptide, wherein
the modified signal peptide includes amino acid residues of X₁X₂X₃X₄X₅X₆ at positions 1 to 6 as counted from the C-terminus, where X₁ is G, P, or A; X₂ is G, S, or A; and X₃ is P, S, or A, provided that X₁X₂X₃ is not GGP; X₄ is A; X₅ is S, H, or F; and X₆ is A and is located at the C-terminus of the modified signal peptide.

In another aspect, the present invention provides an expression vector comprising the nucleic acid construct.

In another aspect, the present invention provides a recombinant *Bacillus* bacterium comprising the nucleic acid construct.

In a further aspect, the present invention provides a method of producing a protein of interest, comprising:
culturing the recombinant *Bacillus* bacterium; and
collecting the protein of interest secreted extracellularly.
In a further aspect, the present invention provides a preproprotein of an antibody-related molecule comprising a modified signal peptide of the present invention and a polypeptide linked to the C-terminal side thereof and encoding the antibody-related molecule, wherein
the modified signal peptide includes amino acid residues of X₁X₂X₃X₄X₅X₆ at positions 1 to 6 as counted from the C-terminus, where X₁ is G, P, or A; X₂ is G, S, or A; and X₃ is P, S, or A, provided that X₁X₂X₃ is not GGP; X₄ is A; X₅ is S, H, or F; and X₆ is A and is located at the C-terminus of the modified signal peptide; and
the amino acid sequence from the N-terminus to position 7 as counted from the C-terminus of the modified signal peptide consists of the amino acid sequence of any of SEQ ID NOs: 7 to 12 or an amino acid sequence having at least 90% identity thereto.

### Detailed Description of the Invention

In the present specification, the term "antibody-related molecule" refers to a protein including a molecular species consisting of an immunoglobulin or a single domain or a combination of two or more domains selected from domains constituting an immunoglobulin. Examples of the domain constituting an immunoglobulin include domains of an immunoglobulin heavy chain, i.e., VH, CH1, CH2, and CH3, and domains of an immunoglobulin light chain, VL and CL. The antibody-related molecule may be a monomer protein or a polymer protein. When the antibody-related molecule is a polymer protein, it may be a homopolymer consisting of a single type of subunits or a heteropolymer consisting of two or more types of subunits. Preferably, the antibody-related molecule is a molecule including an antigen recognition domain of the immunoglobulin and being capable of specifically binding to a target such as a protein. Examples of the antibody-related molecule include immunoglobulins such as IgG and small molecule antibodies such as a Fab, an F(ab')₂, a single chain antibody (scFv), a diabody, and a variable domain of heavy chain of heavy chain antibody (VHH).

In the present specification, the term "VHH" refers to a variable domain of heavy chain of heavy chain antibody. The heavy chain antibody is found in, for example, animals of family Camelidae and cartilaginous fish such as sharks. Incidentally, a heavy chain antibody derived from cartilaginous fish such as sharks is also referred to as VNAR (single variable new antigen receptor domain antibody). The VHH in the present specification is derived from, for example, an animal of family Camelidae or a shark and is preferably derived from an animal of family Camelidae. The VHH is a molecule capable of recognizing an antigen through a single domain and is the smallest unit among the antibody molecules that have been found to date. The VHH produced in the present invention can include one or more variable domains of heavy chain derived from a heavy chain antibody, and the number of the variable domains of heavy chain included in the VHH is not limited. The variable domain of heavy chain included in the VHH produced in the present invention may be a variable domain of heavy chain derived from a natural heavy chain antibody or a modified product thereof. The VHH produced by the present invention may include, for example, a fragment other than the variable domain of heavy chain derived from an immunoglobulin heavy chain (for example, a fragment of the constant region of a heavy chain antibody or a humanderived fragment or a linker sequence) or an amino acid mutation for stabilization.

In the present specification, examples of the animal of family Camelidae include a Bactrian camel, Arabian camel, llama, alpaca, vicuna, and guanaco, and an alpaca is preferable.

In the present specification, the identities of nucleotide sequences and amino acid sequences are calculated by the Lipman-Pearson method (Science, 1985, 227: 1435-1441). Specifically, the identity is calculated by analysis using a homology analysis (Search homology) program of genetic information processing software Genetyx-Win by setting the Unit size to compare (ktup) to 2.

In the present specification, the term "at least 90% identity" relating to an amino acid sequence or a nucleotide sequence refers to an identity of 90% or more and preferably 95% or more.

In the present specification, the term "one or several" that is used relating to deletion, substitution, addition, or insertion of amino acid residues in an amino acid sequence can preferably mean 1 to 5 residues, more preferably 1 to 3 residues, and further preferably 1 or 2 residues, unless otherwise defined. In the present specification, the term "one or several" that is used relating to deletion, substitution, addition, or insertion of nucleotides in a nucleotide sequence can preferably mean 1 to 15 nucleotides, more preferably 1 to 9 nucleotides, and further preferably 1 to 6 nucleotides, unless otherwise defined. In the present specification, the "addition" of an amino acid residue or a nucleotide includes addition of an amino acid residue or a nucleotide to one end or both ends of the sequence.

In the present specification, a "corresponding position" or a "corresponding region" on an amino acid sequence or a nucleotide sequence can be determined by aligning a sequence of interest and a reference sequence (e.g., the amino acid sequence of SEQ ID NO: 1) so as to give a maximum homology (alignment). The alignment of an amino acid sequence or a nucleotide sequence can be carried out using a known algorism, and the procedure thereof is known to those skilled in the art. For example, the alignment can be performed using a Clustal W multiple alignment program (Nucleic Acids Res., 1994, 22: 4673-4680) set to the default. The Clustal W can be used on the website of, for example, European Bioinformatics Institute: EBI [www.ebi.ac.uk/index.html] or DNA data bank of Japan managed by National Institute of Genetics (DDBJ [www.ddbj.nig.ac.jp/searches-j.html]). The position of a sequence of interest aligned at an arbitrary position of the reference sequence by the above-described alignment is regarded as the "corresponding position" to the arbitrary position. In addition, a region between corresponding positions or a region consisting of a corresponding motif is regarded as a corresponding region.

A person skilled in the art can further finely optimize the alignment of the above-obtained amino acid sequence. Such optimum alignment is preferably determined by considering the identity or similarity of amino acid sequences, the frequency of gaps to be inserted, and so on. Here, the identity or similarity of amino acid sequences refers to, when two amino acid sequences are aligned, the rate (%) of the number of positions where the same or similar amino acid residues present in the both aligned two sequences to the number of the full-length amino acid residues. Similar amino acid residues mean that the amino acid residues have similar properties in the polarity or electric charge to each other among 20 amino acids constituting a protein to cause so-called conservative substitution. Groups consisting of such similar amino acid residues are well known to those skilled in the art, and examples thereof include, but not limited to, arginine and lysine; glutamic acid and aspartic acid; serine and threonine; glutamine and asparagine; and leucine and isoleucine.

In the present specification, the term "amino acid residue" means 20 amino acid residues constituting a protein, alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

In the present specification, the term "expression cassette" refers to a nucleic acid construct for controlling the expression of a gene of interest included therein. The expression cassette of the present invention is preferably a DNA construct. In general, an expression cassette contains a code region of the gene of interest to be expressed and a control region for controlling the expression, for example, a promoter. Most control regions are located upstream the coding regions of genes of interest and are operably linked to the coding regions. For example, when the gene of interest is a polynucleotide encoding a preproprotein including a signal peptide, the promoter is located upstream the region encoding the signal peptide and is operably linked to the polynucleotide encoding the preproprotein to control the expression of the preproprotein. In addition, the expression cassette may contain the 3' untranslated region and the 5' untranslated region of the gene of interest. Preferably, the expression cassette includes a restriction enzyme recognition site for allowing insertion and/or resection of the expression cassette to a vector or a genomic DNA at an end thereof. The expression cassette can use for construction of an expression vector or foreign gene introduction into a genomic DNA.

In the present specification, the term "control region" is a region having a function of controlling the expression of the gene (e.g., a region encoding a protein or peptide) located downstream the region. More specifically, the "control region" can be defined as a region existing upstream a coding region of the gene and having a function of controlling the transcription of the coding region by interaction with RNA polymerase. The control region includes a transcription initiation control region and/or a translation initiation control region, or a region from a transcription initiation control region to a translation initiation control region. The transcription initiation control region is a region including a promoter and a transcription initiation point, and the translation initiation control region is a site corresponding to a Shine-Dalgarno (SD) sequence which forms a ribosome binding site together with an initiation codon (Proc. Natl. Acad. Sci. USA, 1974, 71: 1342-1346).

In the present specification, the term "operable linking" of a control region and a polynucleotide of a gene (e.g., a polynucleotide encoding a protein) refers to that a gene and a control region are linked to each other such that the gene can be expressed under the control by the control region. The procedure of "operable linking" of a gene and a control region is well known to those skilled in the art.

In the present specification, the terms "upstream" and "downstream" relating to a gene or a nucleic acid sequence thereof refer to the upstream and downstream, respectively, in the transcription direction of the gene. For example, "a gene located downstream a promoter" means that the gene is present on the 3' side of the promoter in a DNA sense strand, and a region upstream a gene means a region on the 5' side of the gene in a DNA sense strand.

A "signal peptide" has a function of extracellularly secreting a protein of interest operably linked to the peptide. The protein of interest is firstly expressed as a preproprotein having a signal peptide and is then secreted extracellularly through the action of the signal peptide. The preproprotein is secreted extracellularly via cleavage of the signal peptide by a signal peptidase and eventually becomes a mature protein. The signal peptide generally consists of a positively charged N-terminal region, a hydrophobic transmembrane region (H region) forming an α helix, and a C-terminal region having a sheet-like structure (Protein Science, 2012, 21: 13-25). A peptide in the C-terminal region is mainly cleaved by a signal peptidase.

In the present specification, "operable linking" of the amino acid sequence of a protein of interest and a signal peptide refers to the amino acid sequence of a protein of interest being linked to the signal peptide so as to be capable of being secreted extracellularly under the control by the signal peptide. The procedure of the "operable linking" is well known to those skilled in the art, and typically, the amino acid sequence of a protein of interest is linked to the C-terminal side of the signal peptide. Between the amino acid sequence of the protein of interest and the signal peptide that are operable linked to each other, another sequence (e.g., an amino acid sequence that is not secreted extracellularly by processing) may be included.

In the present specification, the term "original" that is used for the function, property, or trait of a cell is used for indicating that the function, property, or trait is naturally present in the cell. In contrast, the term "foreign" is used for indicating a function, property, or trait that is not present originally in the cell and has been exogenously introduced. For example, a "foreign" gene or polynucleotide is a gene or polynucleotide exogenously introduced into the cell. The foreign gene or polynucleotide may be derived from the same organism as the cell into which it is introduced or may be derived from a heterologous organism (i.e., a heterologous gene or polynucleotide).

In the present specification, a secreted protein "has the correct N-terminus" means that at least 50%, 55%, 60%, 65%, 70%, 75%, 80%, 85%, 90%, 95%, 98%, or 100% (all w/w%) of the protein secreted extracellularly via a preproprotein including a signal peptide does not have an amino acid residue that is not originally included at the N-terminal of the protein (e.g., a remaining residue of the signal peptide).

In the present specification, *"Bacillus* bacteria" refers to bacteria of genus *Bacillus,* family *Bacillaceae.* Examples of *Bacillus* bacteria include *Bacillus subtilis, Bacillus cereus, Bacillus thuringiensis, Bacillus megaterium, Bacillus amyloliquefaciens, Bacillus pumilus,* and *Bacillus liqueniformis,* and mutant strains thereof. Incidentally, bacteria of genus *Paenibacillus* or *Brevibacillus* belonging to family *Paenibacillaceae* are not included in *Bacillus* bacteria in the present specification.

The names of genes of *Bacillus subtilis* mentioned in the present specification are described based on *Bacillus subtilis* genome data published on the Internet ([bacillus.genome.ad.jp/], updated on Jan 18, 2006) by JAFAN: Japan Functional Analysis Network for *Bacillus subtilis* (BSORF DB). The gene number of *Bacillus subtilis* mentioned in the present specification is the gene number registered in the BSORF DB.

The names of genes or proteins of antibody-related molecules mentioned in the present specification follow the information registered on Protein Data Bank (PDB) ([www.rcsb.org/]), unless otherwise specified.

The present invention relates to a modified signal peptide and secretory production of a protein of interest using the peptide.

The present invention provides a modified signal peptide. The modified signal peptide provided by the present invention is useful for secretory production of a protein of interest. The modified signal peptide allows efficient extracellular secretion of a protein of interest such as an antibody-related molecule to produce the protein with a high yield. In the antibody-related molecule secreted using the modified signal peptide, almost no unnecessary residue, such as a residue of the signal peptide, remains at the N-terminal, and thus the molecule is valuable as a raw material for various drugs and reagents.

The modified signal peptide of the present invention can be produced by modifying the C-terminal region of a parent signal peptide. Examples of the parent signal peptide include signal peptides that function in *Bacillus* bacteria, and preferred examples thereof include signal peptides derived from *Bacillus* bacteria. Preferred examples of the signal peptide derived from *Bacillus* bacteria include YoaW, lipA, pelB, and YncM signal peptides (SEQ ID NOs: 1 to 4) of *Bacillus subtilis,* the signal peptide (SEQ ID NO: 5) of cellulase derived from *Bacillus* sp. KSM-S237 strain, and the signal peptide (SEQ ID NO: 6) of amylase derived from *Bacillus amyloliquefaciens.* Other preferred examples of the parent signal peptide include signal peptides consisting of amino acid sequences that have at least 90% identity to any of SEQ ID NOs: 1 to 6. Among them, a signal peptide consisting of the amino acid sequence of SEQ ID NO: 4 or 5 or an amino acid sequence having at least 90% identity thereto is preferable as the parent signal peptide.

The C-terminal region of a signal peptide can be estimated by using tertiary structure prediction by CRNPRED ([pdbj.org/crnpred]). The region that is present at the C-terminal of a signal peptide and is predicted not to have an α helix structure can be estimated as the C-terminal region. For example, the C-terminal regions of the above-mentioned parent signal peptides correspond to the 17th to the 24th residues in SEQ ID NO: 1, the 27th to the 32nd residues in SEQ ID NO: 2, the 19th to the 24th residues in SEQ ID NO: 3, the 35th to the 42nd residues in SEQ ID NO: 4, the 24th to the 29th residues in SEQ ID NO: 5, and the 25th to the 31st residues in SEQ ID NO: 6.

Preferably, in the modified signal peptide of the present invention, the C-terminal region is composed of a sequence consisting of six amino acid residues including a modified amino acid residue. More specifically, the modified signal peptide includes amino acid residues of X₁X₂X₃X₄X₅X₆ at positions 1 to 6 as counted from the C-terminus, wherein X₆ is the residue located at the C-terminus of the modified signal peptide, and X₁ is the residue located at position 6 as counted from the C-terminus. The residues of X₁ to X₆ are as follows:
X₁: preferably G, P, or A;
X₂: preferably G, S, or A, more preferably G;
X₃: preferably P, S, or A, more preferably P or S;
X₄: preferably A;
X₅: preferably S, H, or F, more preferably S or H; and
X₆: preferably A.

Preferably, X₁X₂X₃ is not GGP. Preferred examples of the sequence of X₁X₂X₃X₄X₅X₆ include the amino acid sequences of SEQ ID NOs: 13 to 30, more preferably the amino acid sequences of SEQ ID NOs: 14 to 30, and further preferably the amino acid sequences of SEQ ID NOs: 15 and 25 to 28.

The region other than the C-terminal region of the modified signal peptide of the present invention can consist of the same amino acid sequence as that of the parent signal peptide. Alternatively, as long as the function as a signal peptide is not lost, the region other than the C-terminal region of the modified signal peptide may consist of an amino acid sequence having deletion, substitution, addition, or insertion of one or several amino acid residues in the amino acid sequence of the parent signal peptide.

Preferably, the region from the N-terminus to position 7 as counted from the C-terminus of the modified signal peptide of the present invention consists of the amino acid sequence of the region other than the C-terminal region of the signal peptide of *Bacillus* bacteria or an amino acid sequence having at least 90% identity thereto. More preferably, the region from the N-terminus to position 7 as counted from the C-terminus of the modified signal peptide of the present invention consists of the amino acid sequence of any of SEQ ID NOs: 7 to 12 or an amino acid sequence having at least 90% identity thereto. Further preferably, the region from the N-terminus to position 7 as counted from the C-terminus of the modified signal peptide of the present invention consists of the amino acid sequence of SEQ ID NO: 10 or 11 or an amino acid sequence having at least 90% identity thereto.

Accordingly, in a preferred embodiment, the modified signal peptide of the present invention consists of a region consisting of the amino acid sequence of any of SEQ ID NOs: 7 to 12 or an amino acid sequence having at least 90% identity thereto and a region adjacent to the C-terminal side thereof and consisting of X₁X₂X₃X₄X₅X₆. In a more preferred embodiment, the modified signal peptide of the present invention consists of the amino acid sequence of any of SEQ ID NOs: 31 to 35.

The modified signal peptide of the present invention can be produced by modifying the C-terminal region of a parent signal peptide according to a gene engineering process well known in the art. For example, it is possible to construct X₁X₂X₃X₄X₅X₆ by making mutation (deletion, substitution, addition, or insertion) to the individual amino acid residues of the C-terminal region of a parent signal peptide, or the C-terminal region of a parent signal peptide may be substituted with the amino acid sequence of X₁X₂X₃X₄X₅X₆. Alternatively, the region from the N-terminus to position 7 as counted from the C-terminus of a parent signal peptide and X₁X₂X₃X₄X₅X₆ may be linked to each other. On this occasion, as long as the function as a signal peptide is not lost, the amino acid residues of the region from the N-terminus to position 7 as counted from the C-terminus of a parent signal peptide may be mutated.

Preferably, the modified signal peptide can be obtained by preparing a polynucleotide encoding the modified signal peptide of the present invention and expressing it**.** The polynucleotide encoding the modified signal peptide can be prepared by mutating, for example, a polynucleotide encoding a parent signal peptide (hereinafter, referred to as parent gene) so as to encode the modified signal peptide. Alternatively, a nucleotide sequence encoding the modified signal peptide can be chemically synthesized.

The polynucleotide of a parent gene can be prepared by extracting a genomic DNA from, for example, a strain producing a parent signal peptide (e.g., *Bacillus* sp. KSM-S237 strain) by a usual method or by extracting an RNA and synthesizing a cDNA by reverse transcription.

Examples of the method for mutating a parent gene include various mutation technologies known in the art, such as ultraviolet irradiation, site-specific mutagenesis, SOE (splicing by overlap extension)-PCR (Gene, 1989, 77(1): pp. 61-68), and a homologous recombination method. A commercially available kit for site-specific mutagenesis (e.g., QuickChange II Site-Directed Mutagenesis Kit and QuickChange Multi Site-Directed Mutagenesis Kit of Stratagene) can also be used.

Examples of the polynucleotide encoding the modified signal peptide of the present invention include a polynucleotide consisting of the nucleotide sequence of any of SEQ ID NOs: 36 to 41 or a nucleotide sequence having at least 90% identity thereto and a nucleotide sequence linked downstream thereto and encoding X₁X₂X₃X₄X₅X₆. Preferred examples include a polynucleotide consisting of the nucleotide sequence of any of SEQ ID NOs: 36 to 41 or a nucleotide sequence having at least 90% identity thereto and the nucleotide sequence of any of SEQ ID NOs: 42 to 59 linked downstream thereto. More preferred examples include a polynucleotide consisting of the nucleotide sequence of SEQ ID NO: 39 or 40 or a nucleotide sequence having at least 90% identity thereto and the nucleotide sequence of any of SEQ ID NOs: 42 to 59 linked downstream thereto. Further preferred examples include a polynucleotide consisting of the nucleotide sequence of any of SEQ ID NOs: 60 to 64.

The modified signal peptide of the present invention can be used for secreting a protein of interest extracellularly. For example, a polynucleotide encoding a modified signal peptide and a polynucleotide encoding a protein of interest are operably linked to each other, and a preproprotein of the protein of interest including a signal peptide is allowed to be expressed. The protein of interest is secreted extracellularly through the action of the modified signal peptide in the preproprotein.

Accordingly, the present invention provides a nucleic acid construct containing a polynucleotide encoding a protein of interest and a polynucleotide linked upstream of the polynucleotide and encoding the modified signal peptide of the present invention.

Preferably, the nucleic acid construct of the present invention is a nucleic acid construct for expression and extracellular secretion of a protein of interest in a host cell. Preferably, the nucleic acid construct of the present invention is an expression cassette and includes a control region for controlling the expression of the polynucleotide encoding the protein of interest and the polynucleotide encoding the modified signal peptide. In the expression cassette, the polynucleotide encoding the protein of interest and the polynucleotide encoding the modified signal peptide are operably linked to the control region. Preferably, the expression cassette includes a promoter linked upstream of the polynucleotide encoding the modified signal peptide.

Preferred examples of the control region include, but not limited to, a control region that functions in *Bacillus* bacteria, for example, the control region of an α-amylase gene, protease gene, aprE gene, or spoVG gene derived from *Bacillus* bacteria, the control region of a cellulase gene of *Bacillus* sp. KSM-S237 strain (Patent Literature 3), the control region of a cellulase gene of *Bacillus* sp. KSM-64 strain (JP-A-2011-10387), and the control region of a kanamycin resistance gene or chloramphenicol resistance gene derived from *Staphylococcus aureus* (for both, see JP-A-2009-089708). More preferred examples of the control region include promoters derived from *Bacillus* bacteria, for example, the promoter (SEQ ID NO: 65) of a cellulase gene of *Bacillus* sp. KSM-S237 strain, the promoter (SEQ ID NO: 66) of a cellulase gene of *Bacillus* sp. KSM-64 strain, and the promoter (SEQ ID NO: 67) of *Bacillus subtilis* spoVG gene. As preferable control regions, promoters consisting of nucleotide sequences having at least 90% identity to any of SEQ ID NOs: 65 to 67.

The nucleic acid construct of the present invention may contain a nucleic acid sequence other than the polynucleotides encoding the protein of interest and modified signal peptide and the control region, for example, a sequence of a 3' untranslated region or a 5' untranslated region. The nucleic acid construct can include a restriction enzyme recognition site at one end or both ends. The nucleic acid construct of the present invention can be introduced into a vector using the restriction enzyme recognition site. For example, the nucleic acid construct can be introduced into a vector by cleaving the vector with a restriction enzyme and adding thereto the nucleic acid construct of the present invention having a restriction enzyme recognition site at an end.

The species of the protein of interest encoded in the nucleic acid construct of the present invention is not particularly limited. The protein of interest may be a protein that can be originally expressed by the host cell, a heterologous protein, or a protein derived from the same species introduced exogenously. Examples of the protein of interest include an arbitrary protein or an enzyme involved in synthesis of the material of interest, for example, an antibody-related molecule, a cytokine, a hormone, other physiologically active peptides, a transporter, an industrial enzyme such as protease, and a metabolism-associated enzyme.

Preferably, the protein of interest is an antibody-related molecule, more preferably a small molecule antibody such as a Fab, an ScFv, and a VHH. The protein of interest is further preferably selected from the group consisting of a Fab, an ScFv, and a VHH, and is further preferably a VHH. Examples of the Fab, ScFv, and VHH include, but not limited to, those described in Table 1 below.

**[Table 1]**

| **Fab** | | |
|---|---|---|
| Name | Reference | Gene ID |
| 1FDL | Protein data bank | 1FDL |
| 1DQJ | Protein data bank | 1DQJ |
| Ranibizumab | G-SRS | ZL1R02VT79 |

| **ScFv** | | |
|---|---|---|
| Name | Reference | Gene ID |
| 1AZY | Protein data bank | 1AZY |
| Blinatumomab | G-SRS | 4FR53SIF3A |

| **VHH** | | |
|---|---|---|
| Name | Reference | Gene ID |
| 3G9A | Protein data bank | 3G9A |
| 4C57 | Protein data bank | 4C57 |
| 5IMM | Protein data bank | 5IMM |
| 5JQH | Protein data bank | 5JQH |
| 5NBD | Protein data bank | 5NBD |
| 3K1K | Protein data bank | 3K1K |
| 4FHB | Protein data bank | 4FHB |
| 4KSD | Protein data bank | 4KSD |
| 3K81 | Protein data bank | 3K81 |
| 5FV2 | Protein data bank | 5FV2 |
| 4W6W | Protein data bank | 4W6W |
| 5J57 | Protein data bank | 5J57 |
| N15 | Mizukami, M. et al., Protein Expression and Purification 105, 23-32 (2015). | |
| 4GFT | Protein data bank | 4GFT |
| Ozoralizumab | G-SRS | 05ZCK72TXZ |
| Caplacizumab | G-SRS | 2R27AB6766 |
| NbHuI6_VGLW | Vincke, C. et al., J. Biol. Chem., 284, 3273-3284 (2009). | |
| h-NbBcII10_FGLA | Vincke, C. et al., J. Biol. Chem., 284, 3273-3284 (2009). | |
| 1ZVH | Protein data bank | 1ZVH |
| 1ZVY | Protein data bank | 1ZVY |

The polynucleotide encoding the protein of interest included in the nucleic acid construct of the present invention may be codon-optimized in accordance with the species of the host into which the nucleic acid construct is incorporated, as needed. The information on codon usage of various organisms is available from Codon Usage Database ([www.kazusa.or.jp/codon/]).

The nucleic acid construct of the present invention can be used for secretory production of a protein of interest by being introduced into a host cell. The nucleic acid construct may be directly incorporated into the genomic DNA of a host cell or may be incorporated into a vector and then introduced into a host cell. The vector species is not particularly limited and may be an arbitrary vector such as a plasmid, phage, phagemid, cosmid, virus, YAC vector, or shuttle vector. The vector may be a vector that is introduced into the genomic DNA of a host cell or a vector that is retained outside the genomic DNA. A vector that can be replicated inside the host cell is preferable. In one embodiment, the vector into which the nucleic acid construct should be incorporated can be an expression vector, but when the nucleic acid construct to be incorporated is an expression cassette, the vector need not be an expression vector. In one embodiment, the nucleic acid construct of the present invention is an expression cassette including a control region and is incorporated into an arbitrary vector to construct an expression vector. In another embodiment, the expression cassette of the present invention is constructed on an expression vector including a control region by incorporating the nucleic acid construct of the present invention into the expression vector.

Preferred examples of the vector include, but not limited to, shuttle vectors, such as pHA3040SP64, pHSP64R or pASP64 (JP-B-3492935), pHY300PLK (expression vector capable of transforming both *Escherichia coli* and *Bacillus subtilis;* Jpn. J. Genet., 1985, 60: 235-243), and pAC3 (Nucleic Acids Res., 1988, 16: 8732); and plasmids available for transformation of *Bacillus* bacteria, such as pUB110 (J. Bacteriol., 1978, 134: 318-329), pTA10607 (Plasmid, 1987, 18: 8-15), and pHY-S237 (JP-A-2014-158430). Furthermore, a plasmid derived from *Escherichia coli* (e.g., pET22b(+), pBR322, pBR325, pUC57, pUC118, pUC119, pUC18, pUC19, and pBluescript) can also be used.

The species of the host cell is not limited as long as that the signal peptide of the present invention functions so as to secrete a protein of interest inside the host cell. Preferred examples of the host cell include *Bacillus* bacteria, more preferably *Bacillus subtilis* or a mutant strain thereof. Preferred examples of the *Bacillus subtilis* mutant strain that is used as a host cell include extracellular protease-deficient *Bacillus subtilis* strain Dpr9 (see JP-B-4485341). The *Bacillus subtilis* mutant strain to be used as a host cell may have, for example, deletion of a sigma factor such as sigF (see JP-B-4336082) or deletion of recA involved in homologous recombination activity (see JP-B-6088282). A *Bacillus subtilis* mutant strain that is the Dpr9 strain having deletion of one of or both sigF and recA can also be used.

The nucleic acid construct of the present invention or a vector containing it can be introduced into a host cell according to a usual method. For example, a known transformation technique, such as a competent cell method, an electroporation method, a protoplast method, a particle gun method, and PEG method, can be applied to the introduction of the nucleic acid construct or the vector into a host cell. The obtained recombinant cell including the nucleic acid construct of the present invention can be used for secretory production of a protein of interest.

A protein of interest can be produced by culturing a recombinant cell including the nucleic acid construct of the present invention. The recombinant cell may be cultured according to a general culturing method of the host cell. For example, the culture medium for culturing *Bacillus* bacteria includes a carbon source and a nitrogen source necessary for the growth of bacteria. The culture medium may contain other nutrients, such as an inorganic salt, vitamins, and an antibiotic, as needed. The culturing conditions, such as temperature, aerationagitation conditions, culture medium pH, and culturing time, can be appropriately selected according to the bacterial species, trait, culture scale, and so on.

The preproprotein of a protein of interest is expressed intracellularly by culturing the recombinant cell. The preproprotein includes the modified signal peptide of the present invention and a polypeptide linked to the C-terminal side thereof and encoding a protein of interest. In the preproprotein, the modified signal peptide of the present invention is operably linked to a protein of interest, and the protein of interest is secreted extracellularly through the action of the modified signal peptide. Accordingly, in production of a protein of interest by the present invention, there is no need to destroy cells, and the protein of interest secreted extracellularly may be collected. The collected protein of interest can be further purified by using common methods for purifying proteins, such as centrifugation, ammonium sulfate precipitation, gel chromatography, ion exchange chromatography, and affinity chromatography, alone or in appropriate combination.

The protein of interest secreted extracellularly using the modified signal peptide of the present invention has the correct N-terminus, that is, an unnecessary residue, such as a residue of the signal peptide, hardly remains at the N-terminus. Accordingly, the protein of interest produced by the present invention has little variation in the structure and characters resulting from the unnecessary residue, and the function expected in the protein of interest can be well retained. This is very advantageous in proteins of which the activities are affected by a slight difference in the structure, for example, in antibody-related molecules. For example, the antibody-related molecule produced by the present invention has no variation in the activity and is therefore valuable as a raw material for various drugs and reagents.

The present invention also encompasses the following material, production method, use, method, and so on as exemplary embodiments. However, the present invention is not limited to these embodiments.
[1] A nucleic acid construct for expression and extracellular secretion of a protein of interest, comprising a polynucleotide encoding the protein of interest and a polynucleotide linked upstream of the polynucleotide and encoding a modified signal peptide, wherein
   the modified signal peptide includes amino acid residues of X₁X₂X₃X₄X₅X₆ at positions 1 to 6 as counted from the C-terminus, where X₁ is G, P, or A; X₂ is G, S, or A; and X₃ is P, S, or A, provided that X₁X₂X₃ is not GGP; X₄ is A; X₅ is S, H, or F; and X₆ is A and is located at the C-terminus of the modified signal peptide.
[2] The nucleic acid construct according to [1], wherein
   preferably, X₁ is G, P, or A; X₂ is G; X₃ is P or S; X₄ is A, X₅ is S or H; and X₆ is A,
   more preferably, X₁X₂X₃X₄X₅X₆ consists of the amino acid sequence of any of SEQ ID NOs: 13 to 30, and
   further preferably, X₁X₂X₃X₄X₅X₆ consists of the amino acid sequence of any of SEQ ID NOs: 15 and 25 to 28.
[3] The nucleic acid construct according to [1] or [2], wherein the region from the N-terminus to position 7 as counted from the C-terminal of the modified signal peptide:
   preferably consists of the amino acid sequence of a region other than the C-terminal region of the signal peptide of *Bacillus* bacteria or an amino acid sequence having at least 90% identity thereto;
   more preferably consists of the amino acid sequence of any of SEQ ID NOs: 7 to 12 or an amino acid sequence having at least 90% identity thereto; and
   further preferably consists of the amino acid sequence of SEQ ID NO: 10 or 11 or an amino acid sequence having at least 90% identity thereto.
[4] The nucleic acid construct according to [1], wherein the modified signal peptide preferably consists of the amino acid sequence of any of SEQ ID NOs: 31 to 35.
[5] The nucleic acid construct according to any one of [1] to [4], wherein the protein of interest is preferably an antibody-related molecule.
[6] The nucleic acid construct according to [5], wherein
   the antibody-related molecule is preferably selected from the group consisting of a Fab, an ScFv, and a VHH, and
   more preferably a VHH.
[7] The nucleic acid construct according to any one of [1] to [6], preferably further comprising a promoter linked upstream of the polynucleotide encoding the modified signal peptide.
[8] The nucleic acid construct according to [7], wherein
   the promoter is preferably a promoter derived from *Bacillus* bacteria, and
   the promoter more preferably consists of any of SEQ ID NOs: 65 to 67 or a nucleotide sequence having at least 90% identity thereto.
[9] The nucleic acid construct according to any one of [1] to [8], preferably being a nucleic acid construct for expression and extracellular secretion of a protein of interest in a *Bacillus* cell.
[10] An expression vector comprising the nucleic acid construct according to any one of [1] to [9].
[11] A recombinant *Bacillus* bacterium comprising the nucleic acid construct according to any one of [1] to [9].
[12] A method for producing a protein of interest, comprising:
   culturing the recombinant *Bacillus* bacterium according to [11]; and
   collecting the protein of interest secreted extracellularly.
[13] The method according to [12], wherein the protein of interest is preferably an antibody-related molecule.
[14] The method according to [13], wherein the antibody-related molecule is:
   preferably selected from the group consisting of a Fab, an ScFv, and a VHH; and
   more preferably a VHH.
[15] A modified signal peptide comprising amino acid residues of X₁X₂X₃X₄X₅X₆ at positions 1 to 6 as counted from the C-terminus, where X₁ is G, P, or A; X₂ is G, S, or A; and X₃ is P, S, or A provided that X₁X₂X₃ is not GGP; X₄ is A; X₅ is S, H, or F; and X₆ is A and is located at the C-terminus of the modified signal peptide.
[16] The modified signal peptide according to [15], wherein
   preferably, X₁ is G, P, or A; X₂ is G; X₃ is P or S; X₄ is A; X₅ is S or H; and X₆ is A,
   more preferably, X₁X₂X₃X₄X₅X₆ consists of the amino acid sequence of any of SEQ ID NOs: 13 to 30, and
   further preferably, X₁X₂X₃X₄X₅X₆ consists of the amino acid sequence of any of SEQ ID NOs: 15 and 25 to 28.
[17] The modified signal peptide according to [15] or [16], wherein the region from the N-terminus to position 7 as counted from the C-terminus:
   preferably consists of the amino acid sequence of a region other than the C-terminal region of the signal peptide of *Bacillus* bacteria or an amino acid sequence having at least 90% identity thereto;
   more preferably consists of the amino acid sequence of any of SEQ ID NOs: 7 to 12 or an amino acid having at least 90% identity thereto; and
   further preferably consists of the amino acid sequence of SEQ ID NO: 10 or 11 or an amino acid sequence having at least 90% identity thereto.
[18] The modified signal peptide according to [15], preferably consisting of the amino acid sequence of any of SEQ ID NOs: 31 to 35.
[19] The modified signal peptide according to any one of [15] to [18], preferably wherein the modified signal peptide functions in *Bacillus* bacteria.
[20] A preproprotein comprising the modified signal peptide according to any one of [15] to [19] and a polypeptide encoding a protein of interest linked to the C-terminal side thereof.
[21] The preproprotein according to [20], wherein the protein of interest is preferably an antibody-related molecule.
[22] The preproprotein according to [21], wherein the antibody-related molecule is:
   preferably selected from the group consisting of a Fab, an ScFv, and a VHH; and
   more preferably a VHH.
[23] Use of a nucleic acid construct for expression and extracellular secretion of a protein of interest, wherein
   the nucleic acid construct comprises a polynucleotide encoding the protein of interest and a polynucleotide linked upstream of the polynucleotide and encoding a modified signal peptide, and
   the modified signal peptide includes amino acid residues X₁X₂X₃X₄X₅X₆ at positions 1 to 6 as counted from the C-terminus, where X₁ is G, P, or A; X₂ is G, S, or A; and X₃ is P, S, or A, provided that X₁X₂X₃ is not GGP; X₄ is A; X₅ is S, H, or F; and X₆ is A and is located at the C-terminus of the modified signal peptide.
[24] The use according to [23], wherein
   preferably X₁ is G, P, or A; X₂ is G; X₃ is P or S; X₄ is A; X₅ is S or H; and X₆ is A,
   more preferably X₁X₂X₃X₄X₅X₆ consists of the amino acid sequence of any of SEQ ID NOs: 13 to 30, and
   further preferably X₁X₂X₃X₄X₅X₆ consists of the amino acid sequence of any of SEQ ID NOs: 15 and 25 to 28.
[25] The use according to [23] or [24], wherein the region from the N-terminus to position 7 as counted from the C-terminus of the modified signal peptide:
   preferably consists of the amino acid sequence of a region other than the C-terminal region of the signal peptide of *Bacillus* bacteria or an amino acid sequence having at least 90% identity thereto;
   more preferably consists of the amino acid sequence of any of SEQ ID NOs: 7 to 12 or an amino acid sequence having at least 90% identity thereto; and
   further preferably consists of the amino acid sequence of SEQ ID NO: 10 or 11 or an amino acid sequence having at least 90% identity thereto.
[26] The use according to [23], wherein the modified signal peptide preferably consists of the amino acid sequence of any of SEQ ID NOs: 31 to 35.
[27] The use according to any one of [23] to [26], wherein the protein of interest is preferably an antibody-related molecule.
[28] The use according to [27], wherein the antibody-related molecule is:
   preferably selected from the group consisting of a Fab, an ScFv, and a VHH; and
   more preferably a VHH.
[29] The use according to any one of [23] to [28], wherein the nucleic acid construct preferably further comprises a promoter linked upstream of the polynucleotide encoding the modified signal peptide.
[30] The use according to [29], wherein the promoter
   is preferably a promoter derived from *Bacillus* bacteria; and
   more preferably consists of any of SEQ ID NOs: 65 to 67 or a nucleotide sequence having at least 90% identity thereto.
[31] The use according to any one of [1] to [8], wherein the nucleic acid construct is preferably used for expression and extracellular secretion of a protein of interest in a *Bacillus* cell.

### Examples

The present invention will now be more specifically described using examples. However, the technical scope of the present invention is not limited to these examples.

### Material and method

### (1) Host strain

As a host *Bacillus subtilis,* a derivative of *Bacillus subtilis* 168 strain was used. From extracellular protease-deficient *Bacillus subtilis* strain Dpr9 (see JP-B-4485341, extracellular proteases epr, wprA, mpr, nprB, bpr, nprE, vpr, aprE, and aprX are deficient), a sigF gene-deficient strain (Dpr9ΔsigF) was produced according to the method described in JP-B-4336082, and a recA gene-deficient strain (Dpr9ΔrecA) was produced according to the method described in JP-B-6088282. Furthermore, a strain dually deficient in sigF and recA of Dpr9 (Dpr9ΔsigFΔrecA) was produced.

### (2) Culture medium

LB medium: 1% Bacto^{™} Tryptone, 0.5% Bacto^{™} Yeast Extract, and 1% sodium chloride; plate medium containing 1.5% agar; and tetracycline (50 ppm) as needed;
DM3 medium: 1% CMC (Kanto Chemical Co., Inc.), 0.5% Bacto^{™} Casamino Acids, 0.5% Bacto^{™} Yeast Extract, 8.1% disodium succinate·6H₂O, 0.35% dipotassium hydrogen phosphate, 0.15% potassium dihydrogen phosphate, 0.5% glucose, 20 mM magnesium chloride, 0.01% BSA, and 50 ppm tetracycline; plate medium containing 1% agar;
L-mal medium: 1% Bacto^{™} Tryptone, 0.5% Bacto^{™} Yeast Extract, 0.5% sodium chloride, 3.75% maltose monohydrate, 3.75 ppm manganese sulfate, and 15 ppm tetracycline; and
2×L-mal medium: 2% Bacto^{™} Tryptone, 1% Bacto^{™} Yeast Extract, 1% sodium chloride, 7.5% maltose monohydrate, 7.5 ppm manganese sulfate, and 15 ppm tetracycline.

### (3) Reagent

Reagents used were those manufactured by FUJIFILM Wako Pure Chemical Corporation unless otherwise specified.

### Example 1: Construction of plasmid containing modified signal peptide

### (1) Artificial synthesis of gene

The genes of VHHs (1ZVY and 5IMM, which are SEQ ID NOs: 68 and 69, respectively) were artificially synthesized by Thermo Fisher Scientific. Artificially synthesized genes were each attached with scaffold sequences GCAGCTCTTGCAGCA (SEQ ID NO: 70) and TCTATTAAACTAGTT (SEQ ID NO: 71) at the 5' terminus and the 3' terminus, respectively, for inserting the genes into plasmid vectors.

### (2) Construction of plasmid for antibody-related molecule expression

### (Plasmid for S237 signal peptide-1ZVY expression)

A plasmid sequence was amplified using a plasmid pHY-S237 (JP-A-2014-158430) as a template by PCR using a primer set of SEQ ID NOs: 72 and 73 and PrimeSTAR Max DNA polymerase (Takara Bio Inc.). A DNA including the artificially synthesized gene 1ZVY shown in (1) was incorporated into the obtained PCR fragment by In-Fusion HD Cloning Kit (Takara Bio Inc.) to construct a 1ZVY expression plasmid containing the S237 signal peptide.

### (Plasmid for yncM signal peptide-5IMM expression)

An artificially synthesized 5IMM gene was incorporated into pHY-S237 by the same procedure as above. A plasmid sequence was amplified using the obtained plasmid as a template by PCR using a primer set of SEQ ID NOs: 74 and 75 and PrimeSTAR Max DNA polymerase (Takara Bio Inc.). In addition, the DNA of a promoter (p_spoVG) derived from spoVG gene was amplified using the genome of *B. subtilis* 168 strain as a template by PCR using a primer set of SEQ ID NOs: 76 and 77. The DNA of p_spoVG was incorporated into the amplified plasmid fragment by In-Fusion HD Cloning Kit (Takara Bio Inc.).

A plasmid sequence was amplified using the obtained plasmid as a template by PCR using a primer set of SEQ ID NOs: 78 and 79 and PrimeSTAR Max DNA polymerase (Takara Bio Inc.). The DNA of an yncM signal peptide was amplified using the genome of *B. subtilis* 168 strain as a template by PCR using a primer set of SEQ ID NOs: 80 and 81. The DNA of yncM signal peptide was incorporated into the amplified plasmid sequence by In-Fusion HD Cloning Kit (Takara Bio Inc.) to construct a 5IMM expression plasmid including the yncM signal peptide.

Table 2 shows the list of primers used for plasmid construction.

**[Table 2]**

| Primer sequence | SEQ ID NO: |
|---|---|
| 5'-TGCTGCAAGAGCTGCCGGAAATAAA-3' | 72 |
| 5'-TCTATTAAACTAGTTATAGGG-3' | 73 |
| 5'-ATGATGTTAAGAAAGAAAACAAAGC-3' | 74 |
| 5'-CCCGGGAATTCCTGTTATAA-3' | 75 |
| 5'-ACAGGAATTCCCGGGTAAGAAAAGTGATTCTGGGA-3' | 76 |
| 5'-CTTTCTTAACATCATAGTAGTTCACCACCTTTTCC-3' | 77 |
| 5'-CAAGTTCAACTGGTTGAATC-3' | 78 |
| 5'-AGTAGTTCACCTCCTTTTCC-3' | 79 |
| 5'-AGGAGGTGAACTACTATGGCGAAACCACTATCAAA-3' | 80 |
| 5'-AACCAGTTGAACTTGAGCGTCTGCCGCGGGTAAAC-3' | 81 |

### (3) Modification of signal peptide C-terminal region

The C-terminal region of the signal peptide on the plasmid constructed in (2) was modified using PrimeSTAR Mutagenesis Basal Kit (Takara Bio Inc.) according to the manual thereof. The obtained modified signal peptide had a C-terminal region consisting of the sequence of any of SEQ ID NOs: 13 to 30 and 82 to 84 as shown in Table 3 below. The modified plasmid fragment was treated with DpnI and then purified.

### Example 2: Construction of recombinant Bacillus subtilis

Each plasmid was introduced into a host *Bacillus subtilis* by a protoplast method shown below. A glycerol stock of host *Bacillus subtilis* was inoculated in 1 mL of an LB liquid medium and was shake-cultured at 30°C at 210 rpm overnight. On the following day, 10 µL of this culture solution was inoculated in 1 mL of a fresh LB liquid medium and was shake-cultured at 30°C at 210 rpm for about 2 hours. This culture solution was collected in a 1.5-mL tube and was centrifuged at 12,000 rpm for 5 minutes. The supernatant was removed, and the resulting pellet was suspended in 500 µL of SMMP (0.5 M sucrose, 20 mM disodium maleate, 20 mM magnesium chloride hexahydrate, 35% (w/v) Antibiotic medium 3 (Difco Laboratories Ltd.)) containing 4 mg/mL of Lysozyme (Sigma-Aldrich) and was incubated at 37°C for 1 hour. Subsequently, centrifugation was performed at 3,500 rpm for 10 minutes, and the pellet obtained by removing the supernatant was suspended in 400 µL of SMMP. 33 µL of this suspension was mixed with 6 µL of the plasmid fragment constructed in Example 1, and 100 µL of 40% PEG was further added thereto, followed by vortexing. To this solution, 350 µL of SMMP was added, followed by inversion mixing and then shaking at 30°C at 210 rpm for 1 hour. The whole amount thereof was then applied to a DM3 agar medium plate and incubated at 30°C for 2 or 3 days. The produced recombinant *Bacillus subtilis* hosts and the antibody-related molecules (VHH) and signal peptides encoded in the introduced plasmids are shown in Table 3. Incidentally, regarding the plasmid including the unmodified S237 signal peptide, since the Dpr9ΔsigF strain into which the peptide was introduced did not produce a VHH, Dpr9ΔsigFΔrecA strain was used as the hose for improving the productivity.

**[Table 3]**

| Recombinant ID: | Host | VHH | Signal peptide | | |
|---|---|---|---|---|---|
| | | | Species | C-terminal amino acid sequence | SEQ ID NO: |
| Cnt-1 | Dpr9 ΔsigFΔrecA | 1ZVY | Unmodified S237 | Wild-type | |
| 1 | Dpr9 ΔsigF | 1ZVY | Modified S237 | GGAAFA | 13 |
| 2 | | | | AGPASA | 14 |
| 3 | | | | GGSASA | 15 |
| 4 | | | | GGAASA | 16 |
| 5 | | | | GGSAFA | 17 |
| 6 | | | | GGAAHA | 18 |
| 7 | | | | AGSAFA | 19 |
| 8 | | | | AGPAFA | 20 |
| 9 | | | | AGSAHA | 21 |
| 10 | | | | PGSAFA | 22 |
| 11 | | | | GGSAHA | 23 |
| 12 | | | | PGAAFA | 24 |
| 13 | | | | PGSASA | 25 |
| 14 | | | | AGSASA | 26 |
| 15 | | | | AGPAHA | 27 |
| 16 | | | | PGSAHA | 28 |
| 17 | | | | PGAAHA | 29 |
| 18 | | | | PGAASA | 30 |
| 19 | | | | GGPASA | 82 |
| 20 | | | | GGPAHA | 83 |
| 21 | | | | GGPAFA | 84 |
| Cnt-2 | Dpr9 ΔsigF | 5IMM | Unmodified yncM | Wild-type | |
| 22 | Dpr9 ΔsigF | 5IMM | Modified yncM | GGAAFA | 13 |
| 23 | | | | GGSASA | 15 |
| 24 | | | | GGAASA | 16 |
| 25 | | | | GGSAFA | 17 |
| 26 | | | | GGAAHA | 18 |
| 27 | | | | GGSAHA | 23 |

### Test Example 1: Secretory production of VHH using modified S237 signal peptide

### (1) Production amount of VHH

1ZVY-expressing recombinant *Bacillus subtilis* strains (ID: 1 to 21) having the modified S237 signal peptides produced in Example 2 were cultured in a 96-well plate. The protein in each culture supernatant was collected, and the protein amount was quantitatively measured by SDS-PAGE. The procedures of plate culture and protein quantitative measurement will be described in detail later. The averages (N = 3) of the protein amounts in the culture supernatants of the respective recombinant *Bacillus subtilis* are shown in Table 4. Secretion of the VHH from recombinant *Bacillus subtilis* succeeded with 18 out of 21 modified S237 signal peptides.

**[Table 4]**

| Recombinant ID: | Signal peptide C-terminal amino acid sequence | SEQ ID NO: | Protein amount (mg/L) |
|---|---|---|---|
| 1 | GGAAFA | 13 | 26.1 |
| 2 | AGPASA | 14 | 81.1 |
| 3 | GGSASA | 15 | 95.4 |
| 4 | GGAASA | 16 | 97.2 |
| 5 | GGSAFA | 17 | 99.1 |
| 6 | GGAAHA | 18 | 99.4 |
| 7 | AGSAFA | 19 | 104.0 |
| 8 | AGPAFA | 20 | 95.3 |
| 9 | AGSAHA | 21 | 107.0 |
| 10 | PGSAFA | 22 | 111.0 |
| 11 | GGSAHA | 23 | 113.4 |
| 12 | PGAAFA | 24 | 114.9 |
| 13 | PGSASA | 25 | 117.9 |
| 14 | AGSASA | 26 | 93.6 |
| 15 | AGPAHA | 27 | 112.9 |
| 16 | PGSAHA | 28 | 124.5 |
| 17 | PGAAHA | 29 | 122.9 |
| 18 | PGAASA | 30 | 100.4 |
| 19 | GGPASA | 82 | 0.0 |
| 20 | GGPAHA | 83 | 0.0 |
| 21 | GGPAFA | 84 | 0.0 |

### (2) Analysis of N-terminal amino acid of VHH

1ZVY-expressing recombinant *Bacillus subtilis* strains (ID: 13 to 16) having the modified S237 signal peptides produced in Example 2 were flask-cultured. 1ZVY was His-tag purified from each culture supernatant, and the N-terminal amino acid sequence of the obtained 1ZVY was analyzed. The procedures of the flask culture, His-tag purification, and analysis of N-terminal amino acid sequence will be described in detail later. The N-terminal amino acid sequences of 1ZVY secreted in the culture supernatants were all DVQLV, which completely agreed with the N-terminus of the introduced 1ZVY.

### (3) Analysis of N-terminal amino acid of VHH produced using unmodified S237 signal peptide

1ZVY-expressing recombinant *Bacillus subtilis* strain (ID: Cnt-1) having the unmodified S237 signal peptide produced in Example 2 was flask-cultured by the same procedure as in (2). 1ZVY was His-tag purified from the culture supernatant, and the N-terminal amino acid sequence of the obtained 1ZVY was analyzed. The N-terminal amino acid sequence of 1ZVY secreted in the culture supernatant was ADVQL. Accordingly, it was confirmed that unnecessary A derived from the signal peptide remained at the N-terminus of the secreted 1ZVY.

### Test Example 2: Secretory production of VHH using modified yncM signal peptide

### (1) Production amount of VHH

5IMM-expressing recombinant *Bacillus subtilis* strains (ID: 22 to 27) having the modified yncM signal peptides produced in Example 2 were cultured in a 96-well plate. The protein in each culture supernatant was collected, and the protein amount was quantitatively measured by SDS-PAGE. The procedures of plate culture and protein quantitative measurement will be described in detail later. The averages (N = 3) of the protein amounts in the culture supernatants of the respective recombinant *Bacillus subtilis* are shown in Table 5. Secretion of the VHH from recombinant *Bacillus subtilis* succeeded with all 6 modified yncM signal peptides.

**[Table 5]**

| Recombinant ID: | Signal peptide C-terminal amino acid sequence | SEQ ID NO: | Protein amount (mg/L) |
|---|---|---|---|
| 22 | GGAAFA | 13 | 283.4 |
| 23 | GGSASA | 15 | 86.9 |
| 24 | GGAASA | 16 | 218.6 |
| 25 | GGSAFA | 17 | 355.8 |
| 26 | GGAAHA | 18 | 435.0 |
| 27 | GGSAHA | 23 | 72.9 |

### (2) Analysis of N-terminal amino acid of VHH

5IMM-expressing recombinant *Bacillus subtilis* strains (ID: 23) having the modified yncM signal peptide produced in Example 2 was flask-cultured. 5IMM was His-tag purified from the culture supernatant, and the N-terminal amino acid sequence of the obtained 5IMM was analyzed. The procedures of the flask culture, His-tag purification, and analysis of N-terminal amino acid sequence will be described in detail later. The N-terminal amino acid sequence of 5IMM secreted in the culture supernatant was QVQLV, which completely agreed with the N-terminal of the introduced 5IMM.

### (3) Analysis of N-terminal amino acid of VHH produced using unmodified yncM signal peptide

5IMM-expressing recombinant *Bacillus subtilis* strain (ID: Cnt-2) having the unmodified yncM signal peptide produced in Example 2 was flask-cultured by the same procedure as in (2). 5IMM was His-tag purified from the culture supernatant, and the N-terminal amino acid sequence of the obtained 5IMM was analyzed. The N-terminal amino acid sequence of 5IMM secreted in the culture supernatant was TAXLF, and as the residue X at position 3 as counted from the N-terminus, the candidate residue 1 was Q, the candidate residue 2 was D, and the candidate residue 3 was V. When X is V, the sequence agrees with residues TAVLF at positions 23 to 27 of the unmodified yncM signal peptide. Accordingly, it was confirmed that unnecessary amino acid sequence derived from the signal peptide remained at the N-terminus of the secreted 5IMM.

### Reference: Methods used in test examples 1 and 2

### (1) Culture of recombinant Bacillus subtilis (96-well plate culture)

The recombinant *Bacillus subtilis* produced in Example 2 was inoculated in 300 µL of an LB medium containing 50 ppm tetracycline and was shake-cultured at 30°C overnight to prepare a pre-culture solution. The pre-culture solution was inoculated at 1% in 200 µL of an L-mal medium placed in a 96-well plate and was shake-cultured at 30°C for 72 hours. After the culture, 150 µL of the culture solution was centrifuged at 4°C at 3,000 rpm for 10 minutes, and the supernatant was collected.

### (Flask culture)

The recombinant *Bacillus subtilis* produced in Example 2 was inoculated in 1 mL of an LB medium containing 50 ppm tetracycline and was shaken to and fro at 30°C overnight to prepare a pre-culture solution. The pre-culture solution was inoculated at 1% in 20 mL of 2×L-mal medium placed in a baffled conical flask and was shake-cultured at 30°C for 72 hours. After the culture, 1 mL of the culture solution was put in a microtube and centrifuged at 4°C at 15,000 rpm for 5 minutes, and the supernatant was collected.

### (2) SDS-PAGE

The culture supernatant collected in (1) and Laemmli Sample Buffer (Bio-Rad Laboratories, Inc.) were mixed at equal amounts and were then heat-treated at 99°C for 5 minutes to prepare each sample. As the gel, Mini-PROTEIN TGX Stain-Free (Bio-Rad Laboratories, Inc.) was used. 5 µL of each sample was applied to the respective well and was electrophoresed at 210 V for 25 minutes. As the molecular weight marker, Precision Plus protein Unstained standard (Bio-Rad Laboratories, Inc.) was used. The protein bands were detected with ChemiDoc MP Imaging System. The detected protein was quantitatively measured using a calibration curve generated from a lysozyme preparation (Sigma-Aldrich).

### (3) His-tag purification

50 µL of Ni-NTA Agarose (FUJIFILM Wako Pure Chemical Corporation) was added to 1 mL of the culture supernatant collected in (1), followed by inversion mixing with a rotator at 4°C for 30 minutes. After centrifugation at 500g for 5 minutes, the supernatant was removed. The remained precipitate was suspended in 500 µL of Wash Buffer (30 mM imidazole, 0.5 M NaCl, 10 mM Tris, pH 8.0) by vortexing, and after centrifugation at 500g for 5 minutes, the supernatant was removed. This procedure was repeated twice in total. To the remained precipitate, 50 µL of Elution Buffer (500 mM imidazole, 0.5 M NaCl, 10 mM Tris, pH 7.5) was added, followed by inversion mixing with a rotator at 4°C for 30 minutes. After centrifugation at 500g for 5 minutes, the supernatant was transferred to another tube to prepare a His-Tag purified sample.

### (4) N-terminal amino acid analysis

The purified sample obtained in (3) and Laemmli Sample Buffer (Bio-Rad Laboratories, Inc.) were mixed at equal amounts and were then heat-treated at 99°C for 5 minutes. As the gel, Mini-PROTEIN TGX (Bio-Rad Laboratories, Inc.) was used. 10 µL of the sample was applied to the respective well and was electrophoresed at 210 V for 25 minutes. The protein was transferred to a PVDF membrane using Trans-Blot Turbo Mini PVDF Transfer Packs (Bio-Rad Laboratories, Inc.) and Trans-Blot Turbo System (Bio-Rad Laboratories, Inc.) and was stained with Bio-Safe CBB G-250 stain (Bio-Rad Laboratories, Inc.) for 30 minutes. The background was decolored using a 50% methanol solution, the PVDF membrane was thoroughly washed with water, and then the band showed a molecular weight corresponding to that of the VHH was cut out. Using the cut-out band as a specimen, the amino acid sequence of five residues from the N-terminus of the VHH was analyzed. The analysis of an amino acid sequence was consigned to Japan Institute of Leather Research.

The embodiments of the present invention have been described above, but it should be understood that they are not intended to limit the present invention to the particular embodiments described. Various other changes and modifications within the scope of the present invention will be apparent to those skilled in the art. The publications and patent applications cited herein are incorporated by reference as if fully set forth herein.

### ITEMS

The present invention relates to the following items:
1. A nucleic acid construct for expression and extracellular secretion of a protein of interest, comprising:
   a polynucleotide encoding the protein of interest and a polynucleotide linked upstream of the polynucleotide and encoding a modified signal peptide, wherein
   the modified signal peptide includes amino acid residues of X₁X₂X₃X₄X₅X₆ at positions 1 to 6 as counted from the C-terminus, where X₁ is G, P, or A; X₂ is G, S, or A; and X₃ is P, S, or A, provided that X₁X₂X₃ is not GGP; X₄ is A; X₅ is S, H, or F; and X₆ is A and is located at the C-terminus of the modified signal peptide.
2. The nucleic acid construct according to item 1, wherein X₁ is G, P, or A; X₂ is G; X₃ is P or S; X₄ is A; X₅ is S or H; and X₆ is A.
3. The nucleic acid construct according to item 1, wherein X₁X₂X₃X₄X₅X₆ consists of an amino acid sequence of any of SEQ ID NOs: 13 to 30.
4. The nucleic acid construct according to item 1, wherein X₁X₂X₃X₄X₅X₆ consists of an amino acid sequence of any of SEQ ID NOs: 15 and 25 to 28.
5. The nucleic acid construct according to any one of items 1 to 4, wherein a region from the N-terminus to position 7 as counted from the C-terminus of the modified signal peptide consists of an amino acid sequence of any of SEQ ID NOs: 7 to 12 or an amino acid sequence having at least 90% identity thereto.
6. The nucleic acid construct according to item 5, wherein the region from the N-terminus to position 7 as counted from the C-terminus of the modified signal peptide consists of an amino acid sequence of SEQ ID NO: 10 or 11 or an amino acid sequence having at least 90% identity thereto.
7. The nucleic acid construct according to item 1, wherein the modified signal peptide consists of an amino acid sequence of any of SEQ ID NOs: 31 to 35.
8. The nucleic acid construct according to any one of items 1 to 7, wherein the protein of interest is an antibody-related molecule.
9. The nucleic acid construct according to item 8, wherein the antibody-related molecule is selected from the group consisting of a Fab, an ScFv, and a VHH.
10. The nucleic acid construct according to item 8, wherein the antibody-related molecule is a VHH.
11. The nucleic acid construct according to any one of items 1 to 10, further comprising a promoter linked upstream of the polynucleotide encoding the modified signal peptide.
12. The nucleic acid construct according to item 11, wherein the promoter consists of any of SEQ ID NOs: 65 to 67 or a nucleotide sequence having at least 90% identity thereto.
13. An expression vector comprising the nucleic acid construct according to any one of items 1 to 12.
14. A recombinant *Bacillus* bacterium comprising the nucleic acid construct according to any one of items 1 to 12.
15. A method for producing a protein of interest, comprising:
   culturing the recombinant *Bacillus* bacterium according to item 14; and
   collecting the protein of interest secreted extracellularly.
16. A preproprotein of an antibody-related molecule comprising:
   a modified signal peptide and a polypeptide linked of the C-terminal side thereof and encoding the antibody-related molecule, wherein
   the modified signal peptide includes amino acid residues of X₁X₂X₃X₄X₅X₆ at positions 1 to 6 as counted from the C-terminus, where X₁ is G, P, or A; X₂ is G, S, or A; and X₃ is P, S, or A, provided that X₁X₂X₃ is not GGP; X₄ is A; X₅ is S, H, or F; and X₆ is A and is located at the C-terminus of the modified signal peptide, and
   the amino acid sequence from the N-terminus to position 7 as counted from the C-terminus of the modified signal peptide consists of an amino acid sequence of any of SEQ ID NOs: 7 to 12 or an amino acid sequence having at least 90% identity thereto.
17. Use of a nucleic acid construct for expression and extracellular secretion of a protein of interest, wherein
   the nucleic acid construct includes a polynucleotide encoding the protein of interest and a polynucleotide linked upstream of the polynucleotide and encoding a modified signal peptide, and
   the modified signal peptide includes amino acid residues of X₁X₂X₃X₄X₅X₆ at positions 1 to 6 as counted from the C-terminus, where X₁ is G, P, or A; X₂ is G, S, or A; and X₃ is P, S, or A, provided that X₁X₂X₃ is not GGP; X₄ is A; X₅ is S, H, or F; and X₆ is A and is located at the C-terminus of the modified signal peptide.

## Claims

1. A nucleic acid construct for expression and extracellular secretion of a protein of interest, comprising:
a polynucleotide encoding the protein of interest and a polynucleotide linked upstream of the polynucleotide and encoding a modified signal peptide, wherein
the region from position 7 as counted from the C-terminus to the N-terminus of the modified signal peptide consists of the amino acid sequence of SEQ ID NO: 10 or 11 or an amino acid sequence having at least 90% identity thereto;
the modified signal peptide includes amino acid residues of X₁X₂X₃X₄X₅X₆ at positions 1 to 6 as counted from the C-terminus, where X₁ is G, P, or A; X₂ is G; and X₃ is P, S, or A, provided that X₁X₂X₃ is not GGP; X₄ is A; X₅ is S, H, or F; and X₆ is A and is located at the C-terminus of the modified signal peptide; and
the modified signal peptide has a function of extracellular secretion of the protein of interest.

2. The nucleic acid construct according to Claim 1, wherein X₁ is G, P, or A; X₂ is G; X₃ is P or S, provided that X₁X₂X₃ is not GGP; X₄ is A; X₅ is S, H or F; and X₆ is A and is located at the C-terminus of the modified signal peptide.

3. The nucleic acid construct according to Claim 1, wherein X₁X₂X₃X₄X₅X₆ consists of an amino acid sequence of any of SEQ ID NOs: 13 to 30.

4. The nucleic acid construct according to Claim 1, wherein X₁X₂X₃X₄X₅X₆ consists of an amino acid sequence of any of SEQ ID NOs: 15 and 25 to 28.

5. The nucleic acid construct according to Claim 1, wherein the modified signal peptide consists of an amino acid sequence of any of SEQ ID NOs: 31 to 35.

6. The nucleic acid construct according to any one of Claims 1 to 5, wherein the protein of interest is an antibody-related molecule.

7. The nucleic acid construct according to Claim 6, wherein the antibody-related molecule is selected from the group consisting of a Fab, an ScFv, and a VHH.

8. The nucleic acid construct according to Claim 6, wherein the antibody-related molecule is a VHH.

9. The nucleic acid construct according to any one of Claims 1 to 8, further comprising a promoter linked upstream of the polynucleotide encoding the modified signal peptide.

10. The nucleic acid construct according to Claim 9, wherein the promoter consists of any of SEQ ID NOs: 65 to 67 or a nucleotide sequence having at least 90% identity thereto.

11. An expression vector comprising the nucleic acid construct according to any one of Claims 1 to 10.

12. A recombinant *Bacillus* bacterium comprising the nucleic acid construct according to any one of Claims 1 to 10.

13. A method for producing a protein of interest, comprising:
culturing the recombinant *Bacillus* bacterium according to Claim 12; and
collecting the protein of interest secreted extracellularly.

14. A preproprotein of an antibody-related molecule comprising:
a modified signal peptide and a polypeptide linked of the C-terminal side thereof and encoding the antibody-related molecule, wherein
the modified signal peptide includes amino acid residues of X₁X₂X₃X₄X₅X₆ at positions 1 to 6 as counted from the C-terminus, where X₁ is G, P, or A; X₂ is G; and X₃ is P, S, or A, provided that X₁X₂X₃ is not GGP; X₄ is A; X₅ is S, H, or F; and X₆ is A and is located at the C-terminus of the modified signal peptide, and
the amino acid sequence from position 7 as counted from the C-terminus to the N-terminus of the modified signal peptide consists of an amino acid sequence of SEQ ID NOs: 10 or 11 or an amino acid sequence having at least 90% identity thereto; and
the modified signal peptide has a function of extracellular secretion of the polypeptide encoding the antibody-related molecule.
